# EUROPEAN PATENT SPECIFICATION

(11) **EP 0 667 129 B1**
(45) Date of publication and mention of the grant of the patent: **20.05.1998**
(21) Application number: 95101333.3
(22) Date of filing: 24.08.1992
(51) Int. Cl.: A61N 7/00, A61F 2/46

(54) **Improved tool for removal of plastics material**
Werkzeug zur Entfernung von Kunststoff
Instrument amélioré pour enlever de la matière plastique

(30) Priority: 24.08.1991 GB 9118307
(43) Date of publication of application: 16.08.1995
(62) Divisional of application: 92917959.6
(73) Proprietor: YOUNG, Michael John Radley, Ashburton, South Devon TQ13 7JX (GB); BRADNOCK, Brian Robert Denis Peter, Hemel Hempstead, HP3 0PD (GB)
(72) Inventor: YOUNG, Michael John Radley, Ashburton, South Devon TQ13 7JX (GB); BRADNOCK, Brian Robert Denis Peter, Hemel Hempstead, HP3 0PD (GB)
(74) Representative: Gregory, Timothy Mark

(56) References cited:
- WO-A-90/10423
- GB-A- 2 229 660
- US-A- 4 248 232

## Description

The present invention relates to an improved tool for use in removal of plastics material. A tool of this general type is disclosed in our GB 2229660 but further improvements have been discovered. The tool is particularly, but not exclusively, useful in removing plastics cement from such bores in bones as may be used in hip, or other joint, replacements (hereinafter referred to, for convenience, as hip joint replacements).

In a hip joint replacement operation, a metal implant is provided with a long projection which is inserted into a hole drilled in the medulla of the femur and is held firmly in place by means of a plastics cement. On average, such replacements can be expected to last five to ten years. However, due to repetitive shearing forces during daily use, either the bone/cement interface or the cement/metal interface may weaken and the implant will become loose, requiring revision. Sometimes, the metal of the hip replacement may fracture or the plastics components of it may wear out. In these cases, revision is necessary although in most cases the bone/cement interface usually remains quite strong.

In order to revise any loose or damaged implant, all or most of the plastics cement must be removed before inserting a new prosthesis and re-cementing. Removal of the old cement presents a number of difficulties. It is time-consuming and may cause fracturing of the bone. It involves the careful and tedious use of hand tools such as hammers and cement cutting chisels. High speed burrs have been used, but they frequently perforate the bone and make re-cementing more difficult and not so effective.

GB 2229660 discloses a tool which is powered ultrasonically and is provided with a forward facing working surface connected by a plurality of holes to the rear of that surface. The tool may be inserted into the plastics cement filled hole, which cement then melts under the influence of the ultrasonic vibration and passes through the holes to the rear, from where it can be removed following a reverse movement of the tool. However, the bone may be thin.

It is an object of the present invention to provide an improved tool for removal of plastics material such as cement from a bore, particularly one in a bone, which overcomes the above disadvantage.

According to the present invention there is provided a tool for use in removing plastics material from a hole comprising a work surface having a substantially annular cutting edge adapted to contact said material, piezo electric transducer means operatively connected through a work horn to said work surface to cause it to vibrate ultrasonically and thereby to heat locally said plastics material, cavity means adapted to receive said heated plastics material, wherein the work surface comprises a head flange and on a reverse side thereof the substantially annular cutting edge is formed on one side of a conical face angled to the general direction of the tool. The angle is preferably between 20 and 25 degrees.

Preferably, at least the piezo electric transducer means is sealingly encased in a first enclosure of waterproof plastics material and exterior thereof a second enclosure of stainless steel or the like material.

Advantageously, the waterproof plastics material is an acetal plastics material.

This arrangement allows the tool to be autoclaved or otherwise sterilised for use for another patient.

Embodiments of the present invention will now be more particularly described by way of example and with reference to the accompanying drawings, in which: -
FIGURE 1 is a schematic side elevation, shown partially in cross-section, of a tool head embodying the invention;
FIGURE 2 is a cross setional view of a handle incorporating ultrasonic generating means to power the tool head of Figure 1; and
FIGURE 3 shows in cross section a further embodiment of handle for use with the invention.

Referring now to the drawings, there is shown in the Figures a tool comprising a piezo-electric ceramic transducer 1, connected along a longitudinal axis to a coupling horn 2, which in turn is connected along the longitudinal axis to a work horn 3. At the far end of the work horn 3 is a cavity 4 surrounded by an annular cutting edge 5.

As shown in the Figures, the length of the piezo electric ceramic transducer 1 is half a wavelength, the length of the coupling horn 2 is a full wavelength, while the length of the work horn 3 (which includes the annular cutting edge 5) is an integral number of half wavelengths ensuring that the total probe length can penetrate to the required depth. The term "wavelength" is used to represent the wavelength of the ultrasonic wave generated by the piezo electric ceramic transducer in the material concerned. The preferred material for the work horn and annular cutting edge is titanium or an alloy thereof. At an ultrasonic vibrational frequency in the region of 30-35 kHz, the wavelength of the ultrasonic wave in the titanium alloy is in the region of 70-90mm.

It is well known that many common plastics materials will transmit high frequency vibrations without the significant internal losses which would cause bulk heating of the material. It is also known that when the ultrasound is transmitted through two closely contacted components, the interface can experience a considerable heating effect which under the correct circumstances will produce welding. Such heating can also occur at the interface between a vibrating tool or metal component and the plastics material, the heating being sufficient to melt the plastic. The present invention utilises this effect to drill an annular hole into the plastics material.

The plastics cement material used for hip joint replacements is generally a powder of polymethylmethacrylate beads of diameter less than 100 µm held together in situ by a polymerised methyl methacrylate monomer. This material is prone to creep and is susceptible to localised heating on ultrasonic vibration. The property of creep may be utilised in that, during removal of a core of plastics cement material, the existing cement which remains may be forced into improved engagement with fissures or surface imperfections in the bone by virtue of the ultrasonic vibrations imparted to the cement, and thereby stabilise the interface.

At the work surface, the annular cutting edge can be manipulated by the user of the tool to enable the bore diameter to be widened or, by applying pressure to one side of the tool, to create a hole of oval profile.

The present invention is described with reference to removing a plastics cement from a hip joint replacement during revision of the prosthesis. In this case, the hip bone or femur has a blind hole filled with plastics cement which had originally surrounded the prosthesis, but which has a void where the prosthesis used to be.

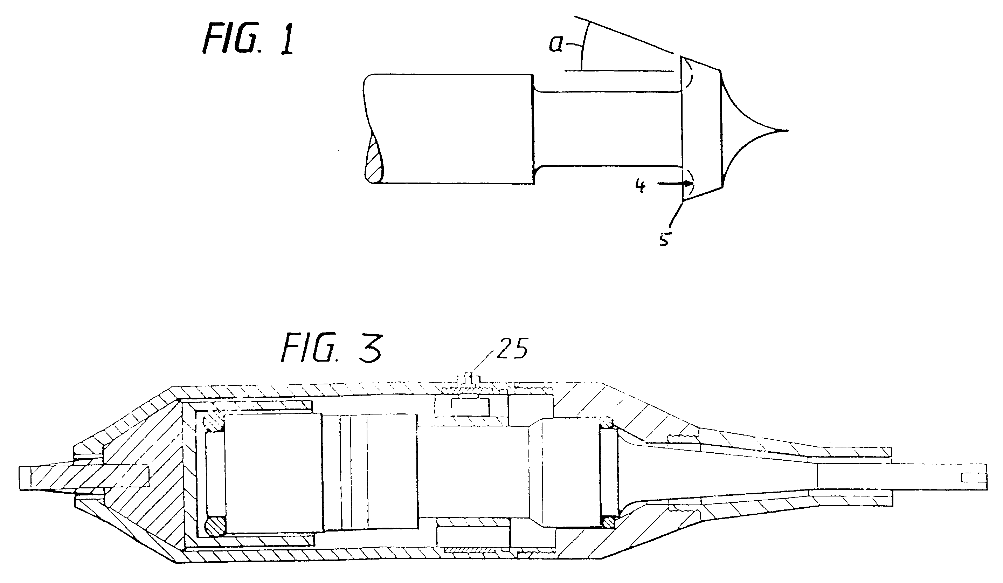

In order to operate the tool, there is shown in Figure 1 an instrument which has a sharp cutting edge on the reverse side of the head flange. The cutting edge is formed on one side of a conical face whole angle "a" critically determines the controlled cutting action of the scraper. There are no connecting holes between front and rear faces of the flange but as the probe is pulled in the reverse direction, it cuts into the cement on one side of the femoral cavity and the removed material collects in the recess behind the flange. This mechanism permits removal of discrete volumes of cement to ensure complete preparation of the endosteal surface ready for recementing.

The optimum angle is between 20° and 25°, this has been found to permit cement removal safely and easily, with the application of only light force. The instrument is of particular use in cases of severe bone resorption which has left areas of extreme weakness in the femur. Great care is required to avoid perforation or fracture of the femur under these circumstances using conventional instruments. This embodiment of the invention achieves this difficult objective without risk. The reverse scraper is also of great use when preparing access to the distal plug and generally precedes the piercing operation effected with a multi-port instrument.

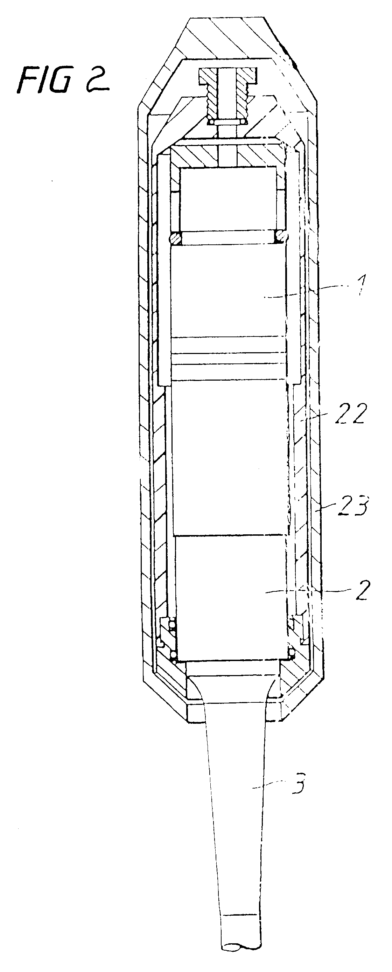

As shown in Figures 2 and 3, the piezo electric transducer part of the tool may be encased, first in a layer of acetal plastics material 22 and then in a layer 23 of stainless steel. This arrangement will allow the tool to be autoclaved or otherwise sterilised in order to permit its use on further patients.

As shown in Figure 3, the handset part of the tool may incorporate a switch 25 and still be autoclavable.

The design philosophy takes account of the conditions prevailing in operating theatres and in particular the strict sterilisation requirements. Several sizes and shapes of oscillatory instrument should ideally be available to the surgeon and a particular case might demand very specialised probe designs. Whereas it is possible to interchange probes on a single handset this procedure is not only inconvenient but results in reduced efficiency of the system if the critical probe/horn interface becomes contaminated with foreign matter. It is desirable therefore to provide a number of independent handsets which can be selected by the surgeon without the need for reconnection or adjustment of switching functions. This dictates the use of a switch incorporated in each handset. Since the handset and cable assembly must be suitable for autoclave sterilisation, the switch assembly requires a special seal design to withstand the temperature and pressure conditions encountered during the sterilisation process.

Figure 3 shows a handset which includes a coaxial switch button operating a sub-miniature micro-switch via a cylindrical moulded seal. The switch is contained in a metal cylindrical sleeve which supports the seal and ensures that it remains water tight even under pressure. This design permits the construction of an oscillatory system offering maximum operating flexibility with inherent reliability. Furthermore there is no need for foot switches which for up to four handsets would involve impractical complications.

## Claims

1. A tool for use in removing plastics material from a hole comprising a work surface having a substantially annular cutting edge (5) adapted to contact said material, piezo electric transducer means (1) operatively connected through a work horn (3) to said work surface to cause it to vibrate ultrasonically and thereby to heat locally said plastics material, cavity means (4) adapted to receive said heated plastics material, characterised in that the work surface comprises a head flange and on a reverse side thereof the substantially annular cutting edge is formed on one side of a conical face angled to the longitudinal direction of the tool .

2. A tool as claimed in Claim 1, characterised in that said angle of the conical face is an angle of between 20 and 25 degrees.

3. A tool as claimed in either Claim 1 or Claim 2, characterised in that at least the piezo electric transducer means is sealingly encased in a first enclosure of waterproof plastics material and exterior thereof a second enclosure of stainless steel or the like material.

4. A tool as claimed in Claim 3, characterised in that the waterproof plastics material is an acetal plastics material.

5. A tool as claimed in either Claim 3 or Claim 4, characterised in that switch means are provided on a handset of said tool, said switch means being sealed to allow the tool to be autoclaved or otherwise sterilised for use for another patient.

## Patentansprüche

1. Werkzeug zum Entfernen von Kunststoffmaterial aus einer Bohrung, umfassend eine Arbeitsfläche mit einer im wesentlichen ringförmigen, zum Kontakt mit dem Material geeigneten Schneidkante (5), eine piezoelektrische Wandlereinheit (1), die über ein Arbeitshorn (3) wirkungsmäßig mit der Arbeitsfläche verbunden ist, um diese in Ultraschallschwingungen zu versetzen und damit das Kunststoffmaterial örtlich zu erhitzen und ein für die Aufnahme des erhitzten Kunststoffmaterials geeignetes Hohlraummittel (4), dadurch gekennzeichnet, daß die Arbeitsfläche einen Kopfflansch umfaßt und daß an einer Rückseite desselben die im wesentlichen ringförmige Schneidkante auf einer Seite einer zur Längsrichtung des Werkzeugs abgewinkelten, konischen Fläche ausgebildet ist.

2. Werkzeug nach Anspruch 1, dadurch gekennzeichnet, daß der Winkel der konischen Fläche ein Winkel von 20 bis 25 Grad ist.

3. Werkzeug nach Anspruch 1 oder 2, dadurch gekennzeichnet, daß mindestens die piezoelektrische Wandlereinheit abgedichtet in einer ersten Ummantelung aus wasserdichtem Kunststoffmaterial und einer an dessen Außenseite vorgesehenen, zweiten Ummantelung aus nichtrostendem Stahl oder dgl. eingekapselt ist.

4. Werkzeug nach Anspruch 3, dadurch gekennzeichnet, daß das wasserdichte Kunststoffmaterial ein Acetalkunststoff ist.

5. Werkzeug nach Anspruch 3 oder 4, dadurch gekennzeichnet, daß an einem Handapparat des Werkzeugs Schaltermittel vorgesehen sind, welche Schaltermittel abgedichtet sind, um eine Autoklavenbehandlung oder anderweitige Sterilisierung des Werkzeugs für Verwendung bei einem anderen Patienten zu gestatten.

## Revendications

1. Instrument destiné à être utilisé pour enlever de la matière plastique d'un trou qui comprend une surface de travail comportant un bord de coupe (5) sensiblement en anneau prévu pour entrer en contact avec ladite matière plastique, un moyen transducteur piézo-électrique (1) relié par un pavillon actif (3) à ladite surface de travail pour la faire vibrer à une fréquence ultrasonore et ainsi chauffer localement ladite matière plastique, un moyen formant cavité (4) prévu pour recevoir ladite matière plastique chauffée, caractérisé en ce que la surface de travail comprend une bride de tête et sur un côté opposé le bord de coupe sensiblement en anneau est formé sur un de ses côtés d'une surface conique faisant un certain angle par rapport à la direction longitudinale de l'instrument.

2. Instrument selon la revendication 1, caractérisé en ce que ledit angle de la surface conique est un angle compris entre 20 et 25 degrés.

3. Instrument selon la revendication 1 ou 2, caractérisé en ce que qu'au moins le moyen transducteur piézo-électrique est logé de façon étanche dans un premier boîtier en matière plastique étanche à l'eau à l'extérieur duquel on trouve un second boitier en acier inoxydable ou en un matériau similaire.

4. Instrument selon la revendication 3, caractérisé en ce que la matière plastique étanche à l'eau est une matière plastique acétalique.

5. Instrument selon la revendication 3 ou 4, caractérisé en ce que des moyens de commutation sont disposés sur une poignée dudit instrument, lesdits moyens de commutation étant fermés hermétiquement pour que l'on puisse passer l'instrument à l'autoclave ou le stériliser par un autre moyen afin de l'utiliser pour un autre patient.
